# EUROPEAN PATENT SPECIFICATION

(11) **EP 1 235 565 B1**
(45) Date of publication and mention of the grant of the patent: **28.12.2005**
(21) Application number: 00988731.6
(22) Date of filing: 20.11.2000
(51) Int. Cl.: A61K 31/135, A61K 31/40, A61K 31/445, A61K 31/155, A61P 1/00

(54) **USE OF 5HT 3 AGONISTS FOR RELAXING THE FUNDUS**
VERWENDUNG VON 5HT3 ANTAGONISTEN ZUR FUNDUSENTSPANNUNG
UTILISATION D'AGONISTES DE 5HT 3 POUR LA RELAXATION DU FOND DE L'ESTOMAC

(30) Priority: 23.11.1999 EP 99203921
(43) Date of publication of application: 04.09.2002
(73) Proprietor: JANSSEN PHARMACEUTICA N.V., 2340 Beerse (BE)
(72) Inventor: MEULEMANS, Ann L. G., Janssen Pharmaceutica N.V., B-2340 Beerse (BE); SCHUURKES, Joannes A. J., Janssen Phamaceut. N.V., B-2340 Beerse (BE)
(86) International application number: PCT/EP2000/011536
(87) International publication number: WO 2001/037824

(56) References cited:
- EP-A- 0 506 545
- EP-A- 0 591 026
- EP-A- 0 666 263
- EP-A- 0 749 966
- FOZARD J R ET AL: "5-HYDROXYTRYPTAMINE: NEW RECEPTORS AND NOVEL DRUGS FOR GASTROINTESTINAL MOTOR DISORDERS" SCANDINAVIAN JOURNAL OF GASTROENTEROLOGY, vol. 25, no. 8, 1 January 1990 (1990-01-01), pages 769-787, XP000571323 ISSN: 0036-5521
- TALLEY N J: "REVIEW ARTICLE: 5-HYDROXYTRYPTAMINE AGONISTS AND ANTAGONISTS IN THE MODULATION OF GASTROINTESTINAL MOTILITY AND SENSATION: CLINICAL IMPLICATIONS" ALIMENTARY PHARMACOLOGY & THERAPEUTICS,GB,BLACKWELL SCIENTIFIC PUBLICATIONS LTD., CAMBRIDGE, vol. 6, no. 3, 1 June 1992 (1992-06-01), pages 273-289, XP000566133 ISSN: 0269-2813

## Description

The present invention is concerned with the use of compounds having 5HT₃ agonistic activity in conditions involving an impaired relaxation of the fundus to food ingestion, in particular dyspepsia.

Dyspepsia is described as a motility disorder. Symptoms can be caused by delayed gastric emptying, an impaired relaxation of the fundus to food ingestion or by hypersensitivity to gastric relaxation. Dyspeptic symptoms are for example a lack of appetite, feeling of fullness, early satiety, nausea, vomiting, bloating and gaseous eructation.

During the consumption of a meal the fundus, *i*.*e*. the proximal part of the stomach, relaxes and provides a "reservoir" function. Patients having an impaired adaptive relaxation of the fundus upon food ingestion have been shown to display dyspeptic symptoms. Therefore, it is believed that compounds which are able to normalize an impaired fundic relaxation are useful to relieve patients suffering from said dyspeptic symptoms.

Patients can have dyspeptic symptoms resulting from a hypercontracted fundus causing a diminished compliance of the stomach and abnormalities in the adaptive fundic relaxation. The "compliance of the stomach" can be expressed as the ratio of the volume of the stomach over the pressure exerted by the stomach wall. The compliance of the stomach relates to the gastric tone, which is the result of the tonic contraction of muscle fibers of the proximal stomach. This proximal part of the stomach, by exerting a regulated tonic contraction (gastric tone), accomplishes the reservoir function of the stomach.

Moreover, also patients with a normal fundic accomodation can have an increased sensitivity to fundic relaxation resulting in similar dyspeptic symptoms.

Patients suffering from early satiety cannot finish a normal meal since they feel saturated before they are able to finish said normal meal. Normally when a subject starts eating, the stomach will show an adaptive relaxation, i. e. the stomach will relax to accept the food that is ingested. This adaptive relaxation is not possible when the compliance of the stomach is hampered which results in an impaired relaxation of the fundus.

EP-0,749,966 discloses fused thiazole derivatives as 5HT₃ agonists useful for the treatment of gastrointestinal tract diseases. EP-0,666,263 discloses condensed thiazole derivates as 5HT₃ agonists useful for the treatment of gastrointestinal disorders and mental disorders. EP-0,591,026 describes 2-[4-(4imidazolyl)piperidino]benzimidazoles that interact with the 5HT₃ and 5HT₄ receptors and useful for treating gastrointestinal disorders, cardiovascular disorders and respiratory problems. EP-0,506,545 discloses the use of 4-amino-1-(2-pyridyl)piperidines as 5-HT₃-agonists for the treatment and prophylaxis of serotoninergic dysfunctions.

Unexpectedly, it was found that compounds having 5HT₃ agonistic activity also have fundic relaxating properties as demonstrated in pharmacological example C.1.

Hence, the use of a compound having 5HT₃ agonistic activity as medicine is provided, and in particular the use of a compound having 5HT₃ agonistic activity for the manufacture of a medicament for restoring an impaired relaxation of the fundus to food ingestion. Both prophylactic and therapeutic treatment are envisaged.

In view of the utility of the compounds having 5HT₃ agonistic activity, it follows that the present invention also provides a method of treating warm-blooded animals, including humans, (generally called herein patients) suffering from impaired relaxation of the fundus to food ingestion. Consequently a method of treatment is provided for relieving patients suffering from conditions, such as, for example, dyspepsia, early satiety, bloating, nausea, vomiting, and gaseous eructation anorexia, caused by an impaired relaxation of the fundus to food ingestion by administration of an effective amount of a 5HT₃ agonistic compound.

Known 5HT₃ agonist are 5-OH-K (5-hydroxykynuramine), 2-methylserotonin, CPBG (1-(m-chlorophenyl)biguanide as described by Kilpatrick G.J. et al. in *Eur. J. Pharmacol.,* 182(1), 193 - 197 (1990)), SR 57277A (4-amino-(6-chloro-2-pyridyl)-1-piperidine hydrochloride as described by Bachy A. et al. in *Eur. J. Pharmacol.,* 237(2-3), 299 - 309 (1993)), and (R)-N-(1-azabicyclo[2.2.2]oct-3-yl)-4,7-dihydro-7-oxothieno[3,2-b]pyridine-6-carboxamide monohydrochloride as described in *Drugs of the Future,* 24(9), 966-968 (1999). The latter compound exhibited an affinity for the 5-HT₃ receptor in canine intestinal smooth muscle of IC₅₀ = 1.3 nM.

### C. Pharmacological examples

### C.1. Gastric tone measured by an electronic barostat in conscious dogs

Gastric tone cannot be measured by manometric methods. Therefore an electronic barostat was used. This allows the study of the physiological pattern and regulation of gastric tone in conscious dogs and the influence of test-compounds on this tone.

The barostat consists of an air injection system which is connected by a double-lumen 14-French polyvinyl tube to an ultrathin flaccid polyethylene bag (maximal volume: ± 700 ml). Variations in gastric tone were measured by recording changes in the volume of air within an intragastric bag, maintained at a constant pressure. The barostat maintains a constant pressure (preselected) within a flaccid air-filled bag introduced into the stomach, changing the volume of air within the bag by an electronic feedback system.

Thus, the barostat measures gastric motor activity (contraction or relaxation) as changes in intragastric volume (decrease or increase resp.) at a constant intragastric pressure. The barostat consists of a strain gauge linked by an electronic relay to an air injection-aspiration system. Both the strain gauge and the injection system are connected by means of double-lumen polyvinyl tube to an ultrathin polyethylene bag. A dial in the barostat allows selection of the pressure level to be maintained within the intragastric bag.

Female beagle dogs, weighing 7-17 kg, were trained to stand quietly in Pavlov frames. They were implanted with a gastric cannula under general anaesthesia and aseptic precautions. After a median laparotomy, an incision was made through the gastric wall in longitudinal direction between the greater and the lesser curve, 2 cm above the nerves of Latarjet. The cannula was secured to the gastric wall by means of a double purse string suture and brought out via a stub wound at the left quadrant of the hypochondrium Dogs were allowed a recovery period of two weeks.

At the beginning of the experiment, the cannula is opened in order to remove any gastric juice or food remnants. If necessary, the stomach is cleansed with 40 to 50 ml lukewarm water. The ultrathin bag of the barostat is positioned into the fundus of the stomach through the gastric cannula. In order to ensure easy unfolding of the intragastric bag during the experiment, a volume of 150-200 ml is injected into the bag by raising the pressure to maximally 14 mm Hg (about 1.87 kPa) very briefly. This procedure is repeated twice.

After a stabilization period of 60 minutes at an intragastric pressure of 6 mmHg (about 0.81 kPa), the test compound was administered subcutaneously, or intraduodenally. Test compounds were screened, i.e. changes in gastric volume are measured, at 0.63 mg/kg s.c. Other doses and routes were tested if a test compound was shown to be active during the screening procedure. Table C-1 summarizes the mean maximal change in volume 1 hour after I.D. or S.C. administration of the compound (R)-N-(1-azabicyclo [2.2.2]oct-3-yl)-4,7-dihydro-7-oxothieno[3,2-b]pyridine-6-carboxamide monohydrochloride at test concentrations of 0.63 mg/kg, 0.16 mg/kg and 0.04 mg/kg.

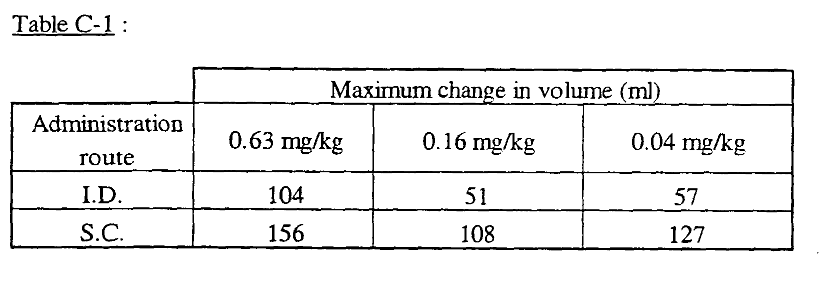

## Claims

1. Use of a compound having 5HT₃ agonistic activity for the manufacture of a medicament for restoring an impaired relaxation of the fundus to food ingestion.

2. Use according to claim 1 wherein an impaired relaxation of the fundus to food ingestion results in fundic hypertension.

3. Use according to claim 1 wherein an impaired relaxation of the fundus to food ingestion results in dyspepsia.

4. Use according to claim 1 wherein an impaired relaxation of the fundus to food ingestion results in lack of appetite, feeling of fullness, early satiety, or bloating.

5. Use according to claim 1 wherein an impaired relaxation of the fundus to food ingestion results in anorexia.

6. Use according to any of claims 1 to 5 wherein the 5HT₃ agonist is 5-hydroxykynuramine, 2-methyl-serotonin or 1-(m-chloro-phenyl)biguanide.

7. Use according to any of claims 1 to 5 wherein the 5HT₃ agonist is 4-amino-(6-chloro-2-pyridyl)-1-piperidine or a salt thereof.

8. Use according to any of claims 1 to 5 wherein the 5HT₃ agonist is (R)-*N*-(1-azabicyclo[2.2.2]oct-3-yl)-4,7-dihydro-7-oxothieno[3,2-b]pyridine-6-carboxamide or a salt thereof.

9. Use according to any of claims 1 to 5 wherein the 5HT₃ agonist has an IC₅₀ value of 1.3 nM or less.

## Patentansprüche

1. Verwendung einer Verbindung mit 5HT₃-agonistischer Wirkung zur Herstellung eines Arzneimittels zur Wiederherstellung einer beeinträchtigten Entspannung des Fundus als Reaktion auf die Aufnahme von Nahrung.

2. Verwendung nach Anspruch 1, wobei eine beeinträchtigte Fundusentspannung als Reaktion auf die Aufnahme von Nahrung zu Fundus-Hypertonie führt.

3. Verwendung nach Anspruch 1, wobei eine beeinträchtigte Fundusentspannung als Reaktion auf die Aufnahme von Nahrung zu Dyspepsie führt.

4. Verwendung nach Anspruch 1, wobei eine beeinträchtigte Fundusentspannung als Reaktion auf die Aufnahme von Nahrung zu Appetitverlust, Völlegefühl, verfrühter Sättigung des Hungergefühls oder zu Blähungen führt.

5. Verwendung nach Anspruch 1, wobei eine beeinträchtigte Fundusentspannung als Reaktion auf die Aufnahme von Nahrung zu Appetitlosigkeit führt.

6. Verwendung nach einem der Ansprüche 1 bis 5, wobei es sich bei dem 5HT₃-Agonisten um 5-Hydroxykynuramin, 2-Methyl-serotonin oder 1-(m-Chlorphenyl)biguanid handelt.

7. Verwendung nach einem der Ansprüche 1 bis 5, wobei es sich bei dem 5HT₃-Agonisten um 4-Amino-(6-chlor-2-pyridyl)-1-piperidin oder eines seiner Salze handelt.

8. Verwendung nach einem der Ansprüche 1 bis 5, wobei es sich bei dem 5HT₃-Agonisten um (R)-N-(1-Azabicyclo[2.2.2]oct-3-yl)-4,7-dihydro-7-oxothieno[3,2-b]pyridin-6-carboxamid oder eines seiner Salze handelt.

9. Verwendung nach einem der Ansprüche 1 bis 5, wobei der 5HT₃-Agonist einen IC₅₀-Wert von 1,3 nM oder darunter aufweist.

## Revendications

1. Utilisation d'un composé ayant une activité d'agoniste des 5HT₃ pour la fabrication d'un médicament destiné à rétablir un relâchement, qui a été altéré, du fundus de l'estomac lors de l'ingestion d'aliments.

2. Utilisation selon la revendication 1, dans laquelle un relâchement altéré du fundus de l'estomac lors de l'ingestion d'aliments conduit à une hypertension fundique.

3. Utilisation selon la revendication 1, dans laquelle un relâchement altéré du fundus de l'estomac lors de l'ingestion d'aliments conduit à une dyspepsie.

4. Utilisation selon la revendication 1, dans laquelle un relâchement altéré du fundus de l'estomac lors de l'ingestion d'aliments conduit à un manque d'appétit, à une sensation d'estomac plein, à une prompte satiété ou à un gonflement.

5. Utilisation selon la revendication 1, dans laquelle un relâchement altéré du fundus de l'estomac lors de l'ingestion d'aliments conduit à une anorexie.

6. Utilisation selon l'une quelconque des revendications 1 à 5, dans laquelle l'agoniste des 5HT₃ est la 5-hydroxy-kynuramine,2-méthyl-sérotonine ou le 1-(m-chloro-phényl)biguanide.

7. Utilisation selon l'une quelconque des revendications 1 à 5, dans laquelle l'agoniste des 5HT₃ est la 4-amino-(6-chloro-2-pyridyl)-1-pipéridine ou un sel de celle-ci.

8. Utilisation selon l'une quelconque des revendications 1 à 5, dans laquelle l'agoniste des 5HT₃ est le (R)-*N*-(1-azabicyclo[2.2.2]oct-3-yl)-4,7-dihydro-7-oxothiéno[3,2-b]pyridine-6-carboxamide ou un sel de celui-ci.

9. Utilisation selon l'une quelconque des revendications 1 à 5, dans laquelle l'agoniste des 5HT₃ a une valeur de la IC₅₀ de 1,3 nM ou moins.
